# EUROPEAN PATENT APPLICATION

(11) **EP 2 407 446 A1**
(43) Date of publication of application: **18.01.2012**
(21) Application number: 10750732.9
(22) Date of filing: 03.03.2010
(51) Int. Cl.: C07C 45/39, B01J 31/06, C07C 49/10, C07B 61/00

(54) **METHOD FOR PRODUCING CARBONYL COMPOUND, CATALYST, AND METHOD FOR PRODUCING CATALYST**

(30) Priority: 12.03.2009 JP 2009059615
(71) Applicant: JX Nippon Oil & Energy Corporation, Chiyoda-ku Tokyo 100-8162 (JP); The University of Tokyo, Bunkyo-Ku Tokyo 113-8654 (JP)
(72) Inventor: KOBAYASHI, Shu, Tokyo 113-8654 (JP); MATSUBARA, Ryosuke, Tokyo 113-8654 (JP); MIYAMURA, Hiroyuki, Tokyo 113-8654 (JP); YAMADA, Jun, Chiyoda-ku Tokyo 100-8162 (JP)
(74) Representative: Grünecker, Kinkeldey, Stockmair & Schwanhäusser
(86) International application number: PCT/JP2010/053455
(87) International publication number: WO 2010/103978

(57) **Abstract**

The method for producing a carbonyl compound according to the invention comprises a step of obtaining a carbonyl compound by oxidation of a secondary alcohol in the presence of a catalyst, wherein the catalyst comprises a carrier obtained by the use of a styrene-based polymer with side chains containing crosslinkable functional groups, wherein the crosslinkable functional groups in the carrier are crosslinked, gold-platinum nanosize clusters supported on the carrier and carbon black supported on the carrier. The production method allows production of a carbonyl compound by oxidation of a secondary alcohol, with high selectivity and a high conversion rate.

## Description

### Technical Field

The present invention relates to a method for producing a carbonyl compound by oxidation of a secondary alcohol, as well as to a catalyst to be suitably used in the production method and a method for producing the catalyst.

### Background Art

Haruta et al., in 1989, have reported that oxygen oxidation reaction using gold nanosize clusters as catalyst has extremely high activity in low temperature carbon monoxide oxidation reaction (Non-patent document 1).

On the other hand, numerous reports describe examples of oxidation reaction of alcohols to aldehydes, ketones and carboxylic acids with metal catalysts using oxygen as the oxidizing agent, wherein a ruthenium or palladium catalyst is used, together with homogeneous catalysts or solid phase catalysts. In recent years, many examples of using gold clusters as catalysts have also been reported (Non-patent document 2).

The present inventors have previously found that by using a microencapsulation method to support transition metal nanosize clusters on a styrene-based polymer, it is possible to produce a very highly active catalyst with palladium or platinum (Non-patent documents 3-4, Patent document 1). There has also been reported production of carbonyl compounds by oxidation reaction with gold catalysts (Patent document 2).

### [Citation List]

### [Patent literature]

[Patent document 1] WO2005/085307
[Patent document 2] Japanese Unexamined Patent Application Publication No. 2007-237116

### [Non-Patent Literature]

.
[Non-patent document 1] J. Catal. 1989, 115, 301-309
[Non-patent document 2] Chem. Rev. 2004, 104, 3037-3058.
[Non-patent document 3] J. Am. Chem. Soc. 2005, 127, 2125-2135.
[Non-patent document 4] Synlett 2005, 813-816.

### Summary of Invention

### [Problems to be Solved by the Invention]

Although numerous examples of using gold clusters as catalysts have been reported to date, problems have been encountered, such as limits on potential substrates and poor selectivity.

It is an object of the present invention to provide a method for producing a carbonyl compound which allows a carbonyl compound to be produced by oxidation of a secondary alcohol, with high selectivity and a high conversion rate, as well as a catalyst to be suitably used in the production method and a method for producing it.

### [Means for Solving the Problems]

The invention provides a method for producing a carbonyl compound that comprises a step of obtaining a carbonyl compound by oxidation of a secondary alcohol in the presence of a catalyst wherein the catalyst comprising:
a carrier obtained by the use of a styrene-based polymer with side chains containing crosslinkable functional groups, wherein the crosslinkable functional groups in the carrier are crosslinked;
gold-platinum nanosize clusters supported on the carrier; and
carbon black supported on the carrier.

In the method for producing a carbonyl compound according to the invention, the styrene-based polymer preferably contains epoxy and hydroxyl groups as the crosslinkable functional groups.

In the method for producing a carbonyl compound of the invention, the aforementioned step is preferably conducted in the absence of a base.

Also, in the method for producing a carbonyl compound of the invention, the styrene-based polymer is preferably a polymer of a polymerizable monomer represented by the following formula (1), a polymerizable monomer represented by the following formula (2), and a polymerizable monomer represented by the following formula (3).

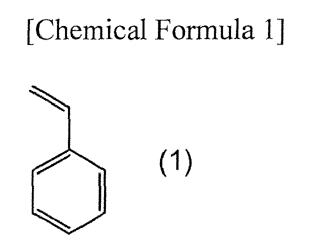

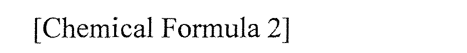

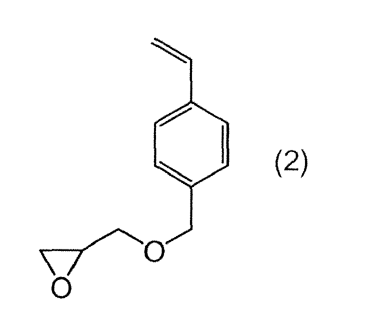

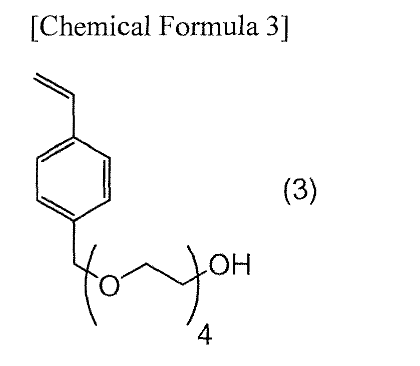

The invention provides a catalyst comprising:
a carrier obtained by a styrene-based polymer with side chains containing crosslinkable functional groups, wherein the crosslinkable functional groups in the carrier are crosslinked;
gold-platinum nanosize clusters supported on the carrier; and
carbon black supported on the carrier.

The invention further provides a method for producing a catalyst, wherein a catalyst comprising a carrier obtained by a styrene-based polymer with side chains containing crosslinkable functional groups, wherein the crosslinkable functional groups in the carrier are crosslinked, gold-platinum nanosize clusters supported on the carrier and carbon black supported on the carrier, is obtained by:
a first step in which a monovalent or trivalent gold compound and a divalent or tetravalent platinum compound is reduced with a reducing agent in a solution comprising a styrene-based polymer with side chains containing crosslinkable functional groups, and carbon black;
a second step in which a poor solvent for the styrene-based polymer is added to the solution for phase separation, to load the gold-platinum nanosize clusters and carbon black on the styrene-based polymer; and
a third step in which the crosslinkable functional groups of the styrene-based polymer are crosslinked after the second step.

In the method for producing a catalyst of the invention, the weight-average molecular weight of the styrene-based polymer is preferably 10,000-150,000.

In the third step of the method for producing a catalyst of the invention, the crosslinkable functional groups of the styrene-based polymer are preferably crosslinked by heating.

In the method for producing a catalyst of the invention, the reducing agent is preferably a boron hydride compound, aluminum hydride compound or silicon hydride compound.

The gold compound in the method for producing a catalyst of the invention is preferably halogenated gold or a halogenated gold-triphenylphosphine complex.

The platinum compound in the method for producing a catalyst of the invention is preferably halogenated platinum or a halogenated platinum-triphenylphosphine complex.

In the method for producing a catalyst of the invention, the gold compound is preferably AuCl(PPh₃) and the platinum compound is preferably Na₂PtCl₆.

### Effects of the Invention

According to the invention, it is possible to provide a method for producing a carbonyl compound which allows a carbonyl compound to be produced by oxidation of a secondary alcohol, with high selectivity and a high conversion rate, as well as a catalyst to be suitably used in the production method and a method for producing it.

### Best Mode for Carrying Out the Invention

Preferred embodiments of the invention will now be described in detail.

The catalyst of the invention comprises:
a carrier by the use of a styrene-based polymer with side chains containing crosslinkable functional groups, wherein the crosslinkable functional groups in the carrier are crosslinked;
gold-platinum nanosize clusters supported on the carrier; and
carbon black supported on the carrier.

In the method for producing a catalyst according to the invention, a catalyst comprising a carrier by the use of a styrene-based polymer with side chains containing crosslinkable functional groups, wherein the crosslinkable functional groups in the carrier are crosslinked, gold-platinum nanosize clusters supported on the carrier, and carbon black supported on the carrier, is obtained by:
a first step in which a monovalent or trivalent gold compound and a divalent or tetravalent platinum compound is reduced with a reducing agent in a solution comprising a styrene-based polymer with side chains containing crosslinkable functional groups, and carbon black;
a second step in which a poor solvent for the styrene-based polymer is added to the solution for phase separation, to load the gold-platinum nanosize clusters and carbon black on the styrene-based polymer; and
a third step in which the crosslinkable functional groups of the styrene-based polymer are crosslinked after the second step.

Loading of the gold-platinum nanosize clusters and carbon black on the styrene-based polymer in the first and second steps is accomplished by a method in which a monovalent or trivalent gold compound and a divalent or tetravalent platinum compound, and the styrene-based polymer and carbon black, a) are dissolved in a suitable polar good solvent and mixed with a reducing agent, and then aggregated with a suitable non-polar poor solvent, or b) are dissolved in an appropriate non-polar or low-polar good solvent and mixed with a reducing agent, and then aggregated with a suitable polar poor solvent. The gold-platinum clusters are loaded by interaction with the aromatic rings of the styrene-based polymer.

The polar good solvent used may be tetrahydrofuran (THF), dioxane, acetone, N,N-dimethylformamide (DMF), N-methyl-2-pyrrolidone (NMP) or the like, and the non-polar or low-polar good solvent may be toluene, dichloromethane, chloroform or the like. The polar poor solvent may be methanol, ethanol, butanol, amyl alcohol or the like, and the non-polar poor solvent may be hexane, heptane, octane or the like.

The polymer concentration, for loading of the gold-platinum clusters on the crosslinkable polymer, will differ depending on the solvent used and the molecular weight of the polymer, but it may be approximately 5.0-200 mg/mL and preferably 10-100 mg/ml. The monovalent or trivalent gold compound is used at 0.01-0.5 mmol and preferably 0.03-0.2 mmol with respect to 1 g of the polymer. The divalent or tetravalent platinum compound is used at 0.01-0.5 mmol and preferably 0.05-0.2 mmol with respect to 1 g of the polymer. The reducing agent may be used at 1-10 equivalents of the amount required for reduction, and for example, when reduction of a monovalent gold compound and a tetravalent platinum compound is to be accomplished with sodium boron hydride, the sodium boron hydride is preferably used at 0.5-5 moles with respect to 1 mol of the gold compound and platinum compound.
The temperature and time necessary for reduction will depend on the type of gold compound, platinum compound and reducing agent, but it will usually be between 0°C-50°C, and preferably room temperature, for 1-24 hours. The poor solvent used for phase separation is added dropwise in a 1-10 times and preferably 2-5 times (v/v) amount with respect to the good solvent, over a period of 0.5-5 hours.

The monovalent or trivalent gold compound is preferably halogenated gold, or a halogenated gold-triphenylphosphine complex. The compound AuCl(PPh₃) is particularly preferred.

Preferred divalent or tetravalent platinum compounds are halogenated platinum and halogenated platinum-triphenylphosphine complex. The compound Na₂PtCl₆ is particularly preferred.

The carbon black may be Ketchen black or the like.

The reducing agent used may be a boron hydride compound, aluminum hydride compound or silicon hydride compound, and is preferably sodium boron hydride or borane.

The styrene-based polymer has a side chain containing a crosslinkable functional group. The crosslinkable functional group preferably includes an epoxy group and a hydroxyl group. The side chain containing a crosslinkable functional group may consist entirely of the crosslinkable functional group, or it may have the crosslinkable functional group bonded to a divalent group.

The divalent groups may be relatively short alkylenes, such as approximately C1-6 alkylene groups, but they preferably have main chains represented by -R¹(OR²)_{w}-, -R¹(COOR²)ₓ- or - R¹(COOR²)_{y}(OR²)_{z}- (wherein R¹ represents a covalent bond or a C1-6, and preferably a covalent bond or a C1-2 alkylene group, each R² independently represents a C2-4 and preferably C2 alkylene group, w, x and z represent integers of 1-10, and y represents 1 or 2), because these are hydrophilic. Preferred divalent groups are -CH₂(OC₂H₄)₄- and - CO(OC₂H₄)₄-.

Examples of such styrene-based polymers include styrene-based polymers obtained by copolymerization of a monomer mixture comprising a monomer having a structure represented by the following formula (4): 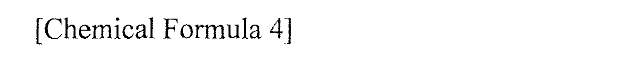 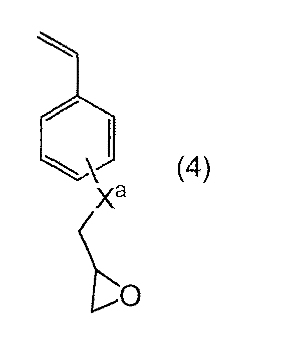 (wherein X^{a} represents an alkylene group or an ether bond-containing alkylene group)
or the following formula (5): 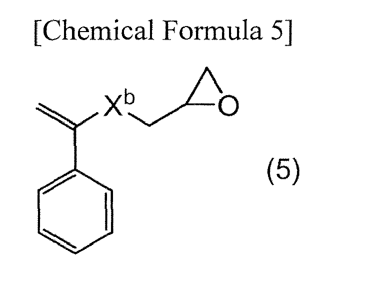 (wherein X^{b} represents an alkylene group or an ether bond-containing alkylene group),
at 5-60% of the total monomer,
a monomer having a structure represented by the following formula (6): 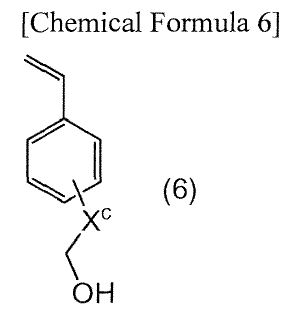 (wherein X^{c} represents an alkylene group or an ether bond-containing alkylene group)
or the following formula (7): 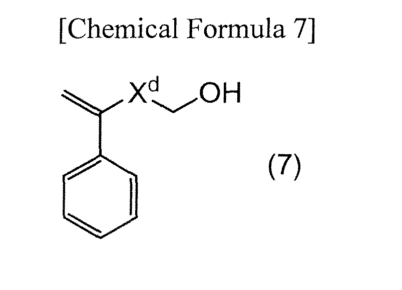 (wherein X^{d} represents an alkylene group or an ether bond-containing alkylene group)
at 10-60% of the total monomer,
the total of these being no greater than 100%, and the remainder consisting of styrene monomer when their total is less than 100%.

Preferred styrene-based polymers include polymers of a polymerizable monomer represented by the following formula (1), a polymerizable monomer represented by the following formula (2), and a polymerizable monomer represented by the following formula (3). 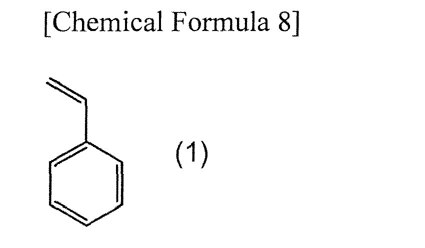 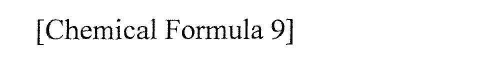 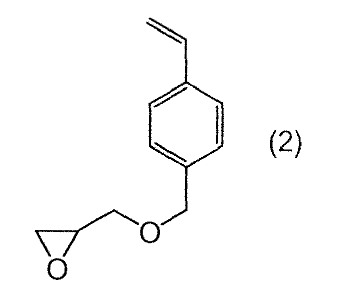 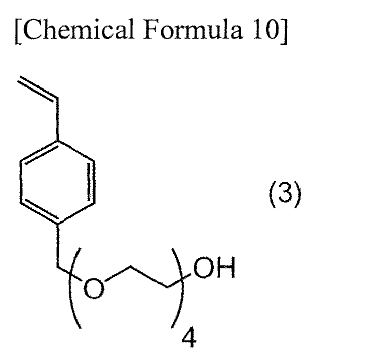 The styrene-based polymer preferably comprises a polymerizable monomer represented by formula (2) at 5-60% and more preferably 10-50% of the total monomer. It also preferably comprises a polymerizable monomer represented by formula (3) at 10-60% and more preferably 20-50% of the total monomer. It preferably comprises polymerizable monomers represented by formula (2) and (3) in a total of less than 100%, with the remainder consisting of styrene monomer represented by formula (1).

The weight-average molecular weight of the styrene-based polymer is preferably between 10,000 and 150,000. The weight-average molecular weight may be measured by gel permeation chromatography (GPC).

When the styrene-based polymer, carbon black, gold compound and platinum compound described above are dissolved in the aforementioned appropriate solvent together with a reducing agent, the gold compound and platinum compound first undergo reduction. When a ligand is bonded to the gold compound and platinum compound, the ligand dissociates. The reduced gold and platinum are taken up into the hydrophobic portion of the polymer as clusters, receiving electron donation from the aromatic ring of the polymer, and becoming stabilized even on the micro level. Next, addition of the poor solvent to the polymer allows phase separation of the styrene-based polymer supporting the gold-platinum clusters and carbon black.

The mean diameter of a single gold-platinum cluster supported on the styrene-based polymer with carbon black is no greater than 20 nm, preferably 0.3-20 nm, more preferably 0.3-10 nm, even more preferably 0.3-5 nm, even yet more preferably 0.3-2 nm and most preferably 0.3-1 nm, and it is believed that numerous gold-platinum clusters are uniformly dispersed in the hydrophobic portions of micelles (the aromatic rings of the styrene-based polymer). Since the metal is in the form of microclusters (small metal blocks), high catalytic activity can be exhibited.

The surrounding environment, including the diameters and valencies of the gold-platinum clusters, can be measured by transmission electron microscope (TEM) or based on expanded X-ray absorption fine-structure (EXAFS).

In the third step, the crosslinkable functional groups of the styrene-based polymer on which the gold-platinum clusters and carbon black are supported as described above are crosslinked. Crosslinking stabilizes the gold-platinum clusters while insolubilizing them with respect to different solvents, thereby allowing leakage of the gold-platinum clusters to be prevented. Crosslinking reaction allows the polymer chains supporting the gold-platinum clusters to be bonded together, and allows bonding to an appropriate carrier, such as a material with crosslinked groups. The crosslinking reaction is conducted by reaction of the crosslinkable functional groups by heating or ultraviolet irradiation, and preferably by heating, under solventless conditions. In addition to these methods, the crosslinking reaction may also be accomplished by a method using a crosslinking agent, a method using a radical polymerization catalyst such as a peroxide or azo compound, a method of adding an acid or base and heating, such as a method of combining a dehydrating condensation agent such as a carbodiimide with an appropriate crosslinking agent, which are methods known in the prior art, for crosslinking of the linear organic polymer compound used.

The temperature for crosslinking of the crosslinkable functional groups by heat will normally be 50-200°C, and is preferably 70-180°C and more preferably 100-160°C. The reaction time for thermal crosslinking reaction will normally be 0.1-100 hours, and is preferably 1-50 hours and more preferably 2-10 hours.

The polymer-supported gold-platinum clusters produced as described above may be prepared as a block or film, or immobilized to the carrier. When crosslinking reaction is carried out between the crosslinkable functional groups (hydroxyl or amino groups, for example) on the surface of a support such as glass, silica gel or a resin and the crosslinkable functional groups of the gold-platinum-containing polymer, the polymer-supported gold-platinum clusters will become firmly immobilized to the support surface. Also, if the crosslinkable functional groups of micelles are used to immobilize the polymer-supported gold-platinum cluster composition onto the surface of a reactor made of a suitable resin or glass, it will be usable as a catalyst-supporting reactor that can be more conveniently reused.

The crosslinked gold-platinum-containing polymer micelles obtained in this manner have numerous pores, and swell with appropriate solvents to exhibit increased surface area. The supported gold and platinum form very small clusters of no greater than several nanometers.

The method for producing a carbonyl compound according to the invention comprises a step of obtaining a carbonyl compound by oxidation of a secondary alcohol in the presence of a catalyst that has a carrier comprising a styrene-based polymer with side chains containing crosslinkable functional groups, wherein the crosslinkable functional groups are crosslinked, and gold-platinum nanosize clusters and carbon black supported on the carrier. Using such a production method allows production of a carbonyl compound by oxidation of a secondary alcohol, with high selectivity and a high conversion rate.

In the method for producing a carbonyl compound of the invention, the aforementioned step is preferably conducted in the absence of a base. According to the method for producing a carbonyl compound of the invention, it is possible to produce a carbonyl compound by oxidation of a secondary alcohol, with high selectivity and a high conversion rate, even in the absence of a base.

When the secondary alcohol substrate is represented by R³R⁴CHOH, R³ and R⁴ may be the same or different and each represents an aliphatic group, an alicyclic aliphatic group or an aromatic group, and R³ and R⁴ may also include heteroatoms. The method for producing a carbonyl compound of the invention is particularly effective when R³ is a methyl group and R⁴ is a C2-6 alkyl group. In this case, R⁴ may be either linear or branched. In particular, if the method for producing a carbonyl compound of the invention is used for oxidation of 2-butanol wherein R³ is a methyl group and R⁴ is an ethyl group, it is possible to produce methyl ethyl ketone with high selectivity and a high conversion rate.

The oxidizing agent used in the method for producing a carbonyl compound of the invention may be oxygen gas or air. The reaction solvent used may be any simple solvent or mixed solvent, so long as it swells the polymer and dissolves the substrate alcohol. A mixed solvent of water and an organic solvent will also be effective in some cases. Organic solvents include benzotrifluoride (BTF) and methyl ethyl ketone. When a mixed solvent is used, the mixing ratio of the water and organic solvent is preferably between 1:1 and 1:10 (volume ratio). The catalyst amount is preferably 0.1-10% (mol/mol) as gold and 0.1-10% (mol/mol) as platinum, with respect to the substrate. The substrate concentration is 0.01-1 mmol/ml and preferably 0.05-0.5 mmol/ml. The reaction temperature is 0-80°C and preferably between room temperature and 60°C, and the reaction time is 1-50 hours.

A base may also be added as an additive for the reaction. In such cases, it is preferred to use an aqueous solution of an alkali metal carbonate or hydroxide salt. The amount of base used is preferably 0.05-3 equivalents with respect to the substrate.

### Examples

The invention will now be illustrated by examples, which are not intended to restrict the invention.
The 4-vinylbenzyl glycidyl ether was synthesized by the method described in Patent document 1. The other compounds used were commercial products, purified as necessary. The yield of the carbonyl compound obtained by the oxidation reaction was quantified by gas chromatography using an internal standard. A GC-17A by Shimadzu Corp. was used as the gas chromatograph.

### <Polymer-supported gold-platinum cluster catalyst Synthesis Example 1>

Sodium hydride (60% in mineral oil, 5.2 g) was added to 150 mL of THF, and then tetraethylene glycol (25.4 g, 131 mmol) was added to the reaction mixture at 0°C. After stirring at room temperature for 1 hour, 1-chloromethyl-4-vinylbenzene (13.3 g, 87.1 mmol) was added and stirring was continued for 12 hours. The mixture was cooled to 0°C, diethyl ether was added, a saturated ammonium chloride aqueous solution was added, and the reaction was suspended. After extracting the aqueous phase with ether, the combined organic phases were dried over anhydrous sodium sulfate and the solvent was distilled off under reduced pressure. The obtained residue was purified by silica gel column chromatography to obtain tetraethyleneglycol mono-2-phenyl-2-propenyl ether (20.6 g, 66.2 mmol, 76%).
¹HNMR (CDCl₃)δ2.55-2.59 (m, 1H), 3.59-3.73 (m, 16H), 4.55 (s, 2H), 5.25 (d, 1H, J = 6.4 Hz), 5.53 (d, 1H, J = 18 Hz), 6.71 (dd, 1H, J = 11.0, 17.9 Hz), 7.22-7.27 (m, 3H), 7.31-7.39 (m, 2H); ¹³CNMR δ61.8, 69.5, 70.5, 70.69, 70.74, 72.6, 73.0, 113.8, 126.3, 128.0, 136.0,137.1,138.0.

After dissolving styrene (2.1 g, 20.2 mmol), 4-vinylbenzyl glycidyl ether (4.0 g, 21.1 mmol), tetraethyleneglycol mono-2-phenyl-2-propenyl ether (6.0 g, 19.4 mmol) and 2,2'-azo(isobutyronitrile) (0.1812 g, 1.28 mmol) in chloroform (11 ml), the solution was subjected to deaeration and then stirred in argon at room temperature for 48 hours. The reaction mixture was cooled to room temperature, and then 11 ml of THF was added and the reaction mixture was slowly added dropwise to 1 1 of ether at 0°C, and the obtained precipitate was separated off by filtration and thoroughly rinsed with methanol. It was then dried under reduced pressure at room temperature to obtain a crosslinked styrene-based polymer (polymer 1) (6.72 g, weight-average molecular weight: 44,664) as a transparent rubber-like solid. The monomer component ratio of the copolymer was determined by ¹H-NMR and found to be styrene: 4-vinylbenzyl glycidyl ether:tetraethyleneglycol mono-2-phenyl-2-propenyl ether = 32:31:37.

Polymer 1 (500 mg) was dissolved in 32 ml of diglyme (special grade, product of Wako Pure Chemical Industries, Ltd.), and 500 mg of carbon black (Carbon ECP, product of Ketchen Black, Intl.) was added to the solution. Sodium boron hydride (80.2 mg) was slowly added to the mixture, and stirred for 10 minutes. There was also slowly added a diglyme solution (8 ml) of AuCl(PPh₃) (special grade product of Wako Pure Chemical Industries, Ltd.) (139 mg) and Na₂PtCl₆ (product of Wako Pure Chemical Industries, Ltd.) (157 mg), and after stirring for 12 hours, the temperature was raised from 0 degrees to room temperature, and then 120 ml of diethyl ether was added dropwise at room temperature. After recovering the obtained solid by filtration, it was rinsed several times with diethyl ether and dried at room temperature, to obtain 0.732 g of a black solid (hereunder referred to as "MC/CB-Au-Pt").

Next, the MC/CB-Au-Pt (0.732 g) was heated at 150°C for 5 hours under solventless conditions, and the produced black solid was filtered and then rinsed with methylene chloride, crushed with a mortar and dried. This procedure yielded 0.712 g of a black powder (hereunder referred to as "PI/CB-Au-Pt").

The gold and platinum contents of the obtained catalyst PI/CB-Au-Pt were quantified by IPC analysis, indicating a gold content of 0.10 mmol/g and a platinum content of 0.14 mmol/g. Using a transmission electron microscope (TEM) (JEM-1200EX II, product of JEOL Corp.), the obtained catalyst PI/CB-Au-Pt was confirmed to have gold-platinum cluster sizes of 4-5 nm.

### [Example 1]

In a round bottom flask there were combined 2-butanol (product of TCI) (18.5 mg, 0.25 mmol), the PI/CB-Au-Pt obtained in Synthesis Example 1 (24.5 mg, 1 mol%), potassium carbonate (104 mg, 0.75 mmol), water (1.5 ml) and benzotrifluoride (special grade, product of Kanto Kagaku Co., Ltd.) (1.5 ml). After stirring at room temperature for 20 hours under an oxygen atmosphere, the catalyst was filtered out and rinsed with water and dichloromethylene (DCM). The aqueous phase was rinsed with dichloromethylene (10 ml). The yield was determined by gas chromatography, using anisole as the internal standard substance. The 2-butanol disappeared, and methyl ethyl ketone was obtained (15.7 mg, 87% yield).

### [Example 2]

Methyl ethyl ketone was obtained (14.2 mg, 79% yield) by oxidation reaction in the same manner as Example 1, except that potassium hydroxide (42.1 mg, 0.75 mmol) was used instead of potassium carbonate, and the stirring time for the reaction mixture was 6 hours.

### [Example 3]

In a round bottom flask there were combined 2-butanol (product of TCI) (18.5 mg, 0.25 mmol), the PI/CB-Au-Pt obtained in Synthesis Example 1 (24.5 mg, 1 mol%), potassium hydroxide (42.1 mg, 0.75 mmol), water (0.45 ml) and 2-butanone (methyl ethyl ketone) (product of TCI) (0.45 ml), as the solvent. After stirring at 60 degrees for 24 hours under an oxygen atmosphere, the catalyst was filtered out and rinsed with water and 2-butanone. The aqueous phase was rinsed with 2-butanone (10 ml). The yield was determined by gas chromatography, using anisole as the internal standard substance. The 2-butanol disappeared, and methyl ethyl ketone was quantitatively obtained.

### [Example 4]

Oxidation reaction was conducted in the same manner as Example 3, except that no potassium hydroxide was added, the amount of water was 0.20 ml and the amount of 2-butanone was 0.20 ml, upon which the 2-butanol disappeared and methyl ethyl ketone was quantitatively obtained.

### [Example 5]

Oxidation reaction was conducted in the same manner as Example 3, except that no potassium hydroxide was added, the amount of water was 0.10 ml, the amount of 2-butanone was 0.10 ml and the reaction time was 12 hours, upon which the 2-butanol disappeared and methyl ethyl ketone was quantitatively obtained.

### [Comparative Example 1]

Oxidation reaction was conducted in the same manner as Example 1, except that PI/Au-Pt was used, no potassium carbonate was added and the stirring time was 8 hours, and methyl ethyl ketone was obtained at a yield of 67%.

### [Example 6]

For this example, the oxidation reaction was conducted using a flow system. A glass column (0.5 cm² × 5.0 cm) by Kyoshin Co., Ltd. was used for the flow system, HPLC (SHIMADZU model, LC-20AD) was used as the pump, and a Teflon^{R} tube was used for the line. A butanone solution (0.114 mmol/ml) of butanol and a potassium hydroxide aqueous solution (0.342 mmol/ml) were passed from top to bottom through a glass column packed with a mixture of PI/CB-Au-Pt (120 mg, 0.095 mmol/g as Au, 0.137 mmol/g as Pt, gold-platinum cluster sizes: 2-3 nm) and 360 mg of Celite, at a flow rate of 0.0070 ml/min, an oxygen gas flow rate of 5 ml/min and a column temperature of 60-62°C. A fraction was taken at each prescribed time point and cooled using a -10°C cooler and ice bath. The methyl ethyl ketone yield was determined by gas chromatography, using anisole as the internal standard substance. The results are shown in Table 1. In Table 1, the percentages of recovered starting materials and yields of methyl ethyl ketone are represented as mean values every 2 hours.

**[Table 1]**

| Time(h) | 0-2 | 2-4 | 4-6 | 6-8 | 8-10 | 10-12 | 12-24 |
|---|---|---|---|---|---|---|---|
| Percentage of recovered starting material (%) | 3 | 2 | 3 | 2 | 3 | 3 | 3 |
| Yield(%) | 90 | 93 | 92 | 92 | 93 | 91 | 90 |

### [Example 7]

Oxidation reaction was conducted in the same manner as Example 6, except that water was used instead of a potassium hydroxide aqueous solution, and the butanone solution of butanol and the water were passed through from bottom to top. The results are shown in Table 2. In Table 2, the proportions of recovered starting materials and yields of methyl ethyl ketone are represented as mean values every 2 hours.

**[Table 2]**

| Time(h) | 0-2 | 2-4 | 4-6 | 6-8 | 8-10 | 10-22 | 22-24 |
|---|---|---|---|---|---|---|---|
| Percentage of recovered starting material (%) | 20 | 22 | 20 | 18 | 19 | 18 | 22 |
| Yield(%) | 83 | 95 | 92 | 89 | 86 | 77 | 96 |

## Claims

1. A method for producing a carbonyl compound comprising a step of obtaining a carbonyl compound by oxidation of a secondary alcohol in the presence of a catalyst,
the catalyst comprising:
a carrier obtained by the use of a styrene-based polymer with side chains containing crosslinkable functional groups, wherein the crosslinkable functional groups in the carrier are crosslinked;
gold-platinum nanosize clusters supported on the carrier; and
carbon black supported on the carrier.

2. The method for producing a carbonyl compound according to claim 1, wherein the styrene-based polymer contains epoxy groups and hydroxyl groups as the crosslinkable functional groups.

3. The method for producing a carbonyl compound according to claim 1 or 2, wherein the step is conducted in the absence of a base.

4. The production method according to any one of claims 1 to 3, wherein the styrene-based polymer is a polymer of a polymerizable monomer represented by the following formula (1), a polymerizable monomer represented by the following formula (2), and a polymerizable monomer represented by the following formula (3).

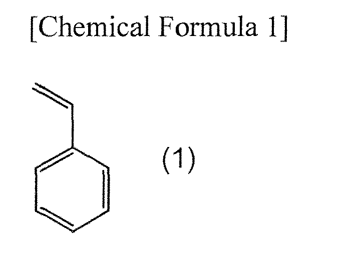

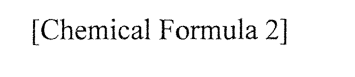

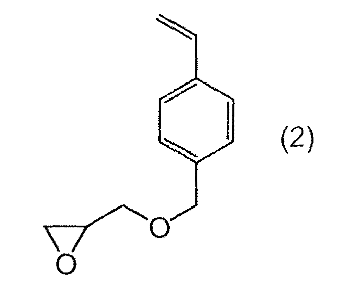

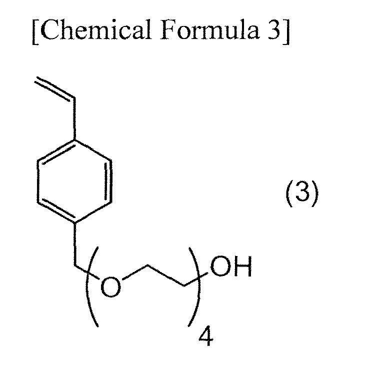

5. A catalyst comprising:
a carrier obtained by the use of a styrene-based polymer with side chains containing crosslinkable functional groups, wherein the crosslinkable functional groups in the carrier are crosslinked;
gold-platinum nanosize clusters supported on the carrier; and
carbon black supported on the carrier.

6. A method for producing a catalyst, wherein a catalyst comprising a carrier obtained by the use of a styrene-based polymer with side chains containing crosslinkable functional groups, wherein the crosslinkable functional groups in the carrier are crosslinked, gold-platinum nanosize clusters supported on the carrier and carbon black supported on the carrier, is obtained by:
a first step in which a monovalent or trivalent gold compound and a divalent or tetravalent platinum compound is reduced with a reducing agent in a solution comprising a styrene-based polymer with side chains containing crosslinkable functional groups, and carbon black;
a second step in which a poor solvent for the styrene-based polymer is added to the solution for phase separation, to load the gold-platinum nanosize clusters and carbon black on the styrene-based polymer; and
a third step in which the crosslinkable functional groups of the styrene-based polymer are crosslinked after the second step.

7. The method for producing a catalyst according to claim 6, wherein the weight-average molecular weight of the styrene-based polymer is 10,000-150,000.

8. The method for producing a catalyst according to claim 6 or 7, wherein in the third step, the crosslinkable functional groups of the styrene-based polymer are crosslinked by heating.

9. The method for producing a catalyst according to any one of claims 6 to 8, wherein the reducing agent is a boron hydride compound, aluminum hydride compound or silicon hydride compound.

10. The method for producing a catalyst according to any one of claims 6 to 9, wherein the gold compound is halogenated gold or a halogenated gold-triphenylphosphine complex.

11. The method for producing a catalyst according to any one of claims 6 to 10, wherein the platinum compound is halogenated platinum or a halogenated platinum-triphenylphosphine complex.

12. The method for producing a catalyst according to any one of claims 6 to 11, wherein the gold compound is AuCl(PPh₃) and the platinum compound is Na₂PtCl₆.
